# EUROPEAN PATENT SPECIFICATION

(11) **EP 3 644 044 B1**
(45) Date of publication and mention of the grant of the patent: **23.12.2020**
(21) Application number: 18202387.9
(22) Date of filing: 24.10.2018
(51) Int. Cl.: G01N 21/64, G02B 21/00, G01N 21/27, G06K 9/00, G06K 9/20, G06K 9/32, G06K 9/44, G06T 5/30, G06T 7/11, G06T 7/155, G06T 7/194, G02B 21/16, G06T 5/00, A61B 5/00, G06T 7/00, H04N 5/235

(54) **CAMERA EXPOSURE CONTROL WHEN ACQUIRING FLUORESCENCE IN SITU HYBRIDIZATION IMAGES**
KAMERABELICHTUNGSSTEUERUNG BEI DER ERFASSUNG VON FLUORESZENZ-IN-SITU-HYBRIDISIERUNGS-BILDERN
COMMANDE D'EXPOSITION DE CAMÉRA LORS DE L'ACQUISITION DES IMAGES D'HYBRIDATION IN SITU EN FLUORESCENCE

(43) Date of publication of application: 29.04.2020
(73) Proprietor: Leica Biosystems Imaging, Inc., Vista, CA 92081-8568 (US)
(72) Inventor: RATCLIFF, Karl, Newcastle-upon-Tyne NE12 8EW (GB)
(74) Representative: Keilitz Haines & Partner Patentanwälte PartGmbB

(56) References cited:
- EP-A1- 3 021 105
- EP-A2- 2 103 925
- WO-A1-98/52018
- WO-A2-2008/019299
- US-A1- 2014 153 811
- US-A1- 2014 205 173

## Description

### FIELD OF THE INVENTION

The present disclosure relates generally to fluorescent *in situ* hybridization (FISH) and more specifically to methods and apparatus for acquiring FISH images.

### BACKGROUND

FISH is a widely used cytogenetic technique based on attaching fluorescent probes to chromosomes to detect, analyze and quantify nuclear abnormalities through detecting the presence of specific sequences of deoxyribonucleic acid (DNA) or ribonucleic acid (RNA). There are many different implementations of FISH, a few of which are:
a. tissue FISH
b. multi-spectral FISH (M-FISH)
c. spot counting FISH
d. flow-fish (i.e. FISH carried out in a flow cytometer)
e. microfluidics-assisted FISH (MA-FISH).

Applications of FISH include diagnosis of several cancer types, analysis of the progression of several cancer types, and identification of chromosomic abnormalities.

Often, as well as acquiring a FISH probe image, i.e. an image that is sensitive to the fluorescent label used to tag the DNA or RNA of interest, a counterstain image is also acquired using a stain such as DAPI (4',6-diamidino-2-phenylindole) to stain the nuclear DNA and hence identify the locations of nuclei in the sample. The FISH probe image and the counterstain image can then be stored together and processed jointly, e.g. for analysis or display.

The following patent publications in the name of Applied Imaging Corporation (now part of Leica Biosystems, Inc.) describe methods and apparatus for acquiring FISH images:
- US 5880473 A
- US 6225636 B1
- US 2002/081014 A1
- US 2003/012420 A1
- WO 98/52018 A1

Known microscopes and other systems that acquire FISH probe images set the exposure of the camera using an auto-exposure technique that relies on image histograms, or other image measurements such as minimum and maximum pixel values, to determine the brightness, i.e. image intensity, of the FISH probe image. The histograms for determining an appropriate exposure are created from the whole FISH probe image or from a selected portion of the FISH probe image. In the latter case, it is known to create the histograms from a portion of the image such as the central portion, or by dividing the whole image into zones and taking a consensus reading from individual brightness values determined for each zone, or by identifying where the FISH signals are located by applying an image threshold and from that creating a mask to mask out areas with below-threshold intensity, so that only the areas with above-threshold intensity are taken account of for the exposure determination. By way of example, a known microscope system with an auto-exposure function for acquiring FISH probe images is CytoVision (RTM) supplied by Leica Biosystems, Inc.

However, it has been observed that FISH probe images acquired with standard auto-exposure setting methods as just described sometimes lack detail. In particular, on closer investigation, it seems that in some cases the portions of the image of interest where the FISH probes are located are not optimally exposed, i.e. either underexposed or overexposed.

WO2008019299A2 discloses an auto-exposure method for capturing acceptably exposed images from all regions of a sample. The method specifically addresses the situation in which the dynamic range of the image sensor and associated electronics is less than the range of intensities of the different regions in the sample, so no single captured image is capable of storing all the signal information. To solve this problem, multiple images are taken. Namely, a first image is taken with a correct exposure for the brightest image areas. However, since areas with dimmer signal will be underexposed in the first image, and perhaps not recorded at all in view of the signal lying outside the dynamic range, a second image is taken at a greater exposure to image these dimmer areas, while at the same time masking the bright areas. The process can be iterated to take further images with successively increasing exposure as desired until the set of images as a whole includes correctly exposed image portions over the whole sample area.

EP3021105A1 discloses another auto-exposure method for capturing acceptably exposed images from all regions of a sample which also addresses the same problem that, when the dynamic range of the image sensor and associated electronics is less than the range of intensities of the different regions in the sample, significant signal from the dim regions may be lost. The method is based on using a pre-defined set of exposure times, e.g. 0.1 seconds, 1 second, 4 seconds and 40 seconds. A first image is acquired with one of these exposure times, which is either the best exposure for the whole image, or an exposure time in the middle of the set. The first image is then processed region-by-region to determine an intensity value for each region. If the intensity value for a particular region is below the detector's saturation value and above the detector's background noise level, then it is determined what the intensity value would be for the longer exposure times in the set. The software then selects the particular exposure time in the set which is predicted to give the highest intensity value that is below saturation. A further image is then taken at that exposure value and the sub-image "tile" covering that particular region is then the one that is used in subsequent image processing. The image ultimately used for the post-processing of the image is thus a composite mosaic of sub-image tiles with different exposures, each tile having the exposure that has been selected for that region.

US 2014/205173 A1 discloses a computer-implemented method for cell-level counting of FISH dots. The count is performed on a composite image obtained by overlaying an image taken with a nuclear counterstain mask with a FISH binary mask. The FISH binary mask is extracted from a FISH image by post-processing using morphological filtering.

### SUMMARY OF THE INVENTION

According to one aspect of the disclosure, there is provided a method of controlling an image acquisition apparatus comprising a fluorescence microscope arrangement to acquire a fluorescence in situ hybridization, FISH, probe image of a sample labeled with a FISH probe. The method comprises:
setting an exposure for acquiring a FISH probe image to an initial value;
acquiring a FISH probe image with the set value of the exposure;
image processing the FISH probe image by applying at least one morphological operator to identify areas of interest around locations of elevated image intensity, and then generating a FISH probe image mask for filtering out all but the areas of interest;
applying the mask to the FISH probe image to determine image intensity in the areas of interest;
determining if the image intensity has a desired intensity value which indicates correct exposure of the FISH probe image, and if 'yes'
then generating and saving a FISH record including a correctly exposed FISH probe image, and if 'no'
then adjusting the exposure value and iterating the method with the adjusted value of the exposure,
wherein the initial exposure value is set so as to acquire an underexposed FISH probe image, and wherein adjusting the exposure value consists of increasing its value, so that the exposure value is successively increased from the initial value as the method iterates.

The mask is used to image process the FISH probe image to determine image intensity within the mask regions, in particular at the cell nuclei. The adjusted value of the exposure for the final image that is saved in the record is then determined based on the image intensities within the mask regions.

The adjusted exposure is determined empirically by successive increases in the exposure until the image intensity is above a desired value, so that the desired value acts as a threshold that needs to be exceeded. Namely, the initial value of the exposure is intentionally set to be too short, so that the initial FISH probe image will be underexposed. By starting the acquisitions with the first FISH probe image being underexposed, the adjusted value of the exposure for the next acquisition will be greater than the initial value. With an initial, intentionally underexposed FISH probe image, the signal in the nuclear regions will almost certainly be too low. From the image intensity in the areas of interest in the underexposed FISH probe image, a higher exposure can be determined which provides sufficiently bright imaging of the FISH signal. In particular, the exposure can be iteratively increased from its initial value, so as to eventually arrive at an exposure which captures a FISH probe image that is sufficiently bright. However, in the case that there are no features, e.g. nuclei, or insufficiently few, which have been fluorescently tagged by FISH, then there can be no signal, or insufficient signal, in the FISH probe image, so the cell areas (i.e. the areas under the mask) will never fluoresce, or not fluoresce strongly enough, and so remain dark, or too dim, regardless of the exposure. Since it cannot be known *a priori* whether there will be any, or sufficient, FISH signal, the incrementing of the exposure is preferably terminated after the iterations result in a maximum exposure value having been reached or approached. This measure avoids unnecessarily repeating the iterative acquisition of FISH probe images when there is no prospect of finding a sufficiently strong FISH signal.

In particular, determining the adjusted value of the exposure comprises acquiring subsequent FISH probe images with exposures successively increased from the initial value and image processing them to determine their image intensities until an exposure is arrived at that provides an image intensity in the areas of interest that is above the desired image intensity. Moreover, as discussed above, it can be useful to terminate the incremental increase of the exposure once a maximum exposure value is reached without obtaining a FISH probe image with an above-threshold image intensity. In this case, the FISH record is generated and saved with one of the FISH probe images with below-threshold image intensity, such as the most recently acquired one, and with metadata, such as a text label in a header of the record, indicating that the FISH probe image most likely has no or insufficient FISH signal.

In theory at least, the inverse of the proposed method based on an underexposed initial FISH probe image would be possible. That is the first FISH probe image could be acquired with intentional overexposure, and the exposure then decremented until a suitable image intensity was reached. However, that would not be as good, since the method would start with the longest exposures, e.g. several seconds, and therefore would take orders of magnitude longer to execute compared with the claimed method, where the initial exposure duration will typically be perhaps a few milliseconds, e.g. 5 ms.

Optionally, the sample may additionally be labeled with a counterstain, in which case the method may further comprise acquiring a counterstain image of the sample.

The counterstain image is then image processed to generate a counterstain image mask that filters out all but areas of, or around, the counterstained areas. The subsequent image processing of the FISH probe image can then be confined to the areas not filtered out by the counterstain image mask, i.e. the FISH probe image processing only takes place on areas of the sample that are counterstained, or areas that are closely related to the counterstained areas, e.g. if a dilation operator is applied to the counterstained pixels for generating the counterstain mask. Applying a dilation operator in this way may be beneficial to help avoid any registration errors between the counterstain image and the FISH probe images causing clipping by the counterstain mask of areas in the FISH probe image where there is FISH signal. With a typical counterstain used for FISH, the counterstained feature locations will be cell nucleus locations, since the counterstain will attach to the nuclear material in the sample.

A further aspect of the invention provides a computer program product for controlling an image acquisition apparatus to acquire a fluorescence *in situ* hybridization, FISH, probe image of a sample labeled with a FISH probe and optionally also a counterstain, the computer program product bearing machine readable instructions for performing the above described methods of controlling an image acquisition apparatus.

A still further aspect of the invention relates to an image acquisition apparatus for acquiring a fluorescence in situ hybridization, FISH, probe image of a sample labeled with a FISH probe. The image acquisition apparatus comprises a fluorescence microscope arrangement operable to acquire FISH probe images of a sample; and a control computer configured to determine appropriate exposures for acquiring FISH probe images with the fluorescence microscope arrangement. The control computer determines an appropriate exposure by:
setting an exposure for acquiring a FISH probe image to an initial value;
acquiring a FISH probe image with the set value of the exposure;
image processing the FISH probe image by applying at least one morphological operator to identify areas of interest around locations of elevated image intensity, and then generating a FISH probe image mask for filtering out all but the areas of interest;
applying the mask to the FISH probe image to determine image intensity in the areas of interest;
determining if the image intensity has a desired intensity value which indicates correct exposure of the FISH probe image, and if 'yes'
then generating and saving a FISH record including a correctly exposed FISH probe image, and if 'no'
then adjusting the exposure value and iterating the method with the adjusted value of the exposure,
wherein the initial exposure value is set so as to acquire an underexposed FISH probe image, and wherein adjusting the exposure value consists of increasing its value, so that the exposure value is successively increased from the initial value as the method iterates.

The apparatus may further comprise: a display; and a display output operable to access the FISH record and transmit the FISH probe image, and optionally the counterstain image, to the display such that the FISH probe image, and optionally also the counterstain image, are displayed.

A still further aspect of the invention relates to a clinical network comprising:
a computer;
a data repository configured to store FISH records, the FISH records including FISH probe images, and optionally counterstain images;
network connections enabling transfer of the FISH records or parts thereof between the computer and the data repository; and
an image acquisition apparatus according to the above operable to generate FISH records and save them to the data repository.

### BRIEF DESCRIPTION OF THE DRAWINGS

In the following, the present invention will further be described by way of example only with reference to exemplary embodiments illustrated in the figures.
Figure 1 is a schematic drawing of an example fluorescence microscope system suitable for acquiring FISH images.
Figure 2 is a flow diagram showing steps involved in performing a method according to an embodiment of the invention.
Figure 3A shows a FISH probe image which was taken without benefit of the invention.
Figure 3B shows a FISH probe image of the same sample as Figure 3A taken with an optimized exposure according to an embodiment of the invention.
Figure 4 shows an example computer network which can be used in conjunction with embodiments of the invention.
Figure 5 is a block diagram of a computing apparatus that may be used for carrying out the image processing needed to implement the invention.

### DETAILED DESCRIPTION OF THE PREFERRED EMBODIMENTS

In the following detailed description, for purposes of explanation and not limitation, specific details are set forth in order to provide a better understanding of the present disclosure. It will be apparent to one skilled in the art that the present disclosure may be practiced in other embodiments that depart from these specific details.

In this document we use the term morphological operator. This term is understood to mean a mathematical operator used for shape analysis, and in particular for extracting image components that are useful in the representation and description of shape. For FISH probe images, this means the shape of cells or cell elements, such as nuclei. Example morphological operators are: dilation, erosion, opening, closing. Further detail can be found in:
- Chapter 9, entitled "Morphological Image Processing", of the text book Gonzalez and Woods "Digital Image Processing" 3rd edition (2008), pp. 627 to 688, ISBN 013168728, and
- Chapter 14, entitled "Edge Detection in Microscope Images", of the text book "Image Analysis in Histology" Edited by Richard Wootton, David Springall and Julia Polak (1995), pp. 241-261, ISBN 0521434823.

### MICROSCOPE PLATFORM

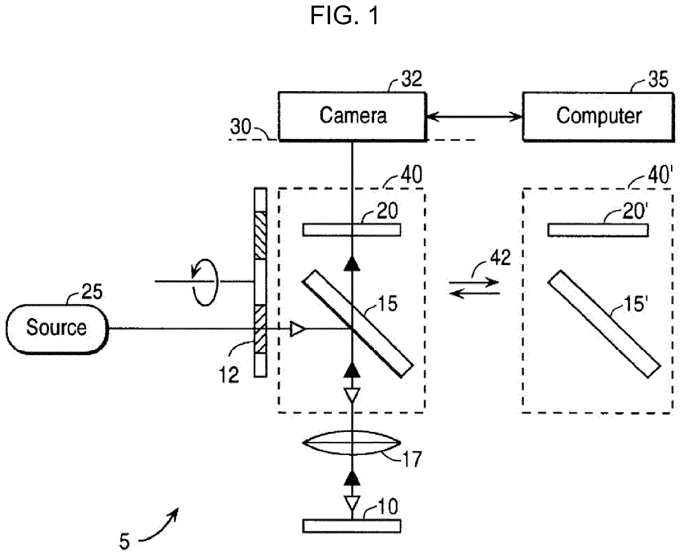

Figure 1 is a schematic drawing of an example reflection-mode (i.e. epi) fluorescence microscope system 5 that is suitable for acquiring FISH images of a sample 10. The optical arrangement in the microscope system 5 includes an excitation filter 12 (shown as one of several such filters on a filter wheel), a dichroic mirror 15, a microscope objective 17 (say 60x to 100x image capture magnification), and an emission filter 20 (sometimes also referred to as a barrier filter). Excitation light from a source 25 passes through the excitation filter 12, is in part reflected by the dichroic mirror 15 and that part proceeds through the microscope objective 17 to the sample 10. The excitation light traveling towards the sample is shown schematically by hollow arrowheads. Fluorescent radiation emitted from the sample 10 passes back through the objective 17, through the dichroic mirror 15, and through the emission filter 20 to form an image in an image plane 30. The fluorescent light traveling away from the sample is shown schematically by solid black arrowheads. The image is digitized by a camera 32, such as one having a charged-coupled device detector, and the digitized image is sent to a computer 35 for subsequent processing.
If the filters have single passbands, the particular filters and dichroic mirror are specific to a single dye in the sample. Images for other dyes in the sample are acquired by substituting optical elements configured for the excitation and emission bands for each other dye. The dichroic mirror and the emission filter are typically rigidly mounted to a supporting structure 40 (shown in phantom), often referred to as a cube, with multiple cubes being movable into and out of the optical path. Oppositely directed arrows 42 represent a suitable mechanism such as a rotatable turret or a detented slide mechanism. The multiple excitation filters are typically deployed on a rotatable filter wheel (as shown). The system may be for monochrome image acquisition or color image acquisition. For color acquisition the CCD camera is a color CCD camera, multiband excitation is provided in three color bands with respective sources, or a broadband source and appropriate filters, and three corresponding emission filters are provided that only transmit within one of three emission bands. Example fluorescent dyes that are used for FISH include: DAPI, FITC (fluorescein isothiocyanate) and cyanine dyes such as Cy3, Cy3.5, Cy5, Cy5.5, and Cy7.

### IMAGE ACQUISITION

The image acquisition of the FISH probe images includes applying a camera auto-exposure method. The auto-exposure method is conceived to set the camera exposure to a suitable value for obtaining a FISH probe image so that the FISH signals are clear and visible, provided that the sample is capable of producing a FISH signal of sufficient brightness. In particular, the proposed method is robust against the presence of bright, highly fluorescent debris which would otherwise result in underexposure of the cell nucleus areas where the FISH fluorescence signal is located.

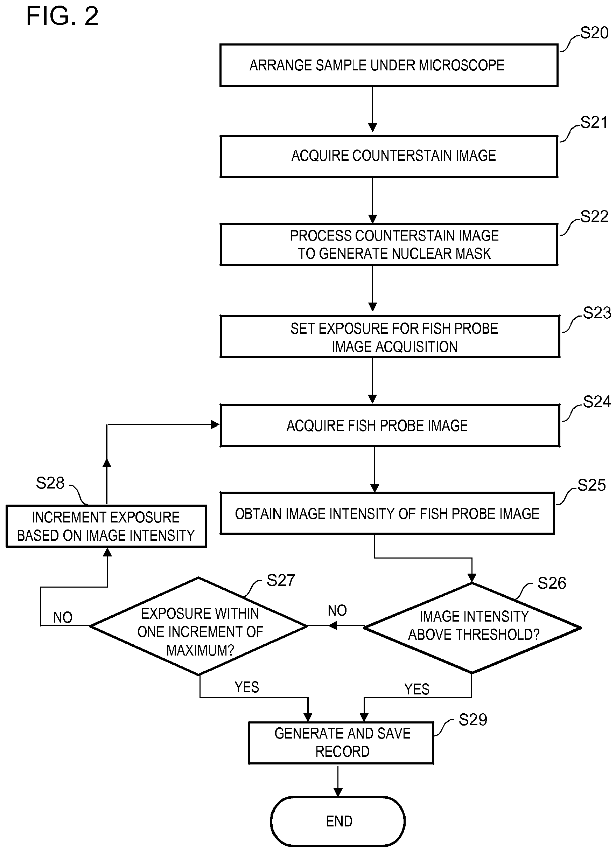

Figure 2 is a flow diagram showing steps involved in performing a method embodying the proposed auto-exposure method.

First, as shown in Step S20 it is of course necessary to provide a sample, e.g. on a slide. The sample has been labeled with a FISH probe and a suitable counterstain. The sample is loaded under the microscope, or other suitable apparatus platform with microscope imaging capability such as a suitably equipped flow cytometer or microfluidic system. The counterstain typically highlights the cell nuclei, but in principle the counterstain could additionally or instead stain other cell features such as cytoplasm or cytoplasmic membrane. In the following we assume a nuclear counterstain.

In Step S21, the method acquires a counterstain image of the sample and saves it.

In Step S22, the method image processes the counterstain image to generate a mask defining areas of interest, i.e. the areas of the nuclei. This may be done, e.g. by applying an image intensity threshold, to produce a mask identifying the locations of the cell nuclei. For example, a closed contour can be determined around each contiguous group of pixels identified to correspond to a nucleus. The closed contours could be used directly for the mask, or the contours could be further processed, e.g. by dilation. Another alternative would be to use some kind of blob analysis to define the mask. Some amount of dilation or other expansion of the nuclear areas may be advantageous to allow for registration errors between the counterstain image and the FISH probe images which are subsequently analyzed using the mask. Namely, if the mask exactly corresponds to the stained nuclear pixels in the counterstain image, then any registration error will result in some proportion of the FISH signal being excluded from the determination of image intensity, and hence make the measured image intensity lower than the true image intensity.

In Step S23, the method sets the exposure of the image acquisition apparatus to an initial value which is expected to result in an underexposure of the FISH probe image.

In Step S24, the method acquires a FISH probe image.

In Step S25, the method image processes the FISH probe image by applying at least one morphological operator to identify areas of interest around locations of elevated image intensity, and then generating a FISH probe image mask around the areas of interest, so that the mask filters out all but the areas of interest. The counterstain mask generated in Step S22 assists the image processing in Step S25 by allowing the image processing to be confined to the areas of interest as defined by the counterstain mask, thereby reducing the amount of processing required and avoiding processing of artefacts not lying in areas of cell material of interest.

### EXAMPLE COMPUTATION FOR STEP S25 (FISH PROBE IMAGE MASK GENERATION)

a. The FISH probe image is filtered by the counterstain mask to set the intensity value of all pixels to zero that are masked out by the counterstain mask.
b. A median filter is applied for noise reduction.
c. A white top hat filter is applied to remove any background ambient light component of the FISH signal preserving image intensity features that are brighter than the background and objects that are smaller than a certain size, wherein the size is chosen so as not to reject any features that are likely to occur with the current image capture magnification (or with a range of likely image capture magnifications).
d. A regional maximum operator is applied to determine the locations of maxima within the FISH probe image. A regional maxima can be defined as a contiguous set of connected pixels, each pixel having an intensity value above a threshold, whose external boundary pixels all have a value less than the threshold.
e. The regional maxima are dilated by applying a dilation operator to avoid the mask filtering out too much.
f. The FISH probe image mask is generated by applying a threshold filter to the image, with the threshold being set so that pixels with above-threshold intensities correspond to the locations of FISH signals.

In Step S26 to S28, the method adjusts the exposure as necessary based on the image intensity in the areas of interest. If the image intensity measurements indicate that brightness in the areas of interest is below a threshold value, i.e. too dark, the method increments the exposure to E' = E + ΔE unless the increased exposure value would exceed a maximum permitted value (test of Step S27). If the maximum exposure is not exceeded, the process flow proceeds from Step S27 to Step S28 and then further to Step S24 and so on to obtain another FISH probe image with the increased exposure. If the maximum exposure would be exceeded by the increment, as tested for in Step S27, this indicates that there is no or insufficient FISH signal to measure, since the longest permitted exposure has still resulted in a FISH probe image which is too dark at the positions where the nuclei are located, in which case the method can end. On the other hand, if the intensity measurements indicate the brightness is high enough, then the exposure was OK, and the iterative part of the method can end after generating and saving a record in Step S29.

In other words, Step S26 tests whether the image intensity obtained in Step S25 has a desired intensity value, which indicates correct exposure of the FISH probe image. If 'yes', then the method can terminate by generating and saving a FISH record including a correctly exposed FISH probe image. It is also sensible to save the counterstain image into the record, although not essential. If'no' then it is tested in Step S27 whether it is sensible to increment the exposure value.

In Step S27, if the incremented exposure value would be higher than a maximum exposure value, then this means in all probability that the sample is not capable of emitting a sufficiently strong FISH signal, so there is no point in iterating further. The method is therefore terminated by saving a record containing, for example, the most recently acquired FISH image. In this case it is also sensible, although not essential, to add some metadata, such as a text label in the record header, stating or indicating that the FISH probe image most likely has no significant or insufficient FISH signal, i.e. this is most likely a failed acquisition.

If Step S27 permits the exposure to be incremented, then this is done in Step S28, and the process flow returns to Step S24 to acquire a further FISH probe image.

It is envisaged that adjusting the exposure to find an appropriate value will comprise repeatedly acquiring FISH probe images with incrementally increasing exposures by repeated traversal of the loop of Steps S24 to S28, with image processing determining at each iteration the FISH image intensity at the areas of interest, e.g. cell nuclei, until an exposure is arrived at that provides an image intensity which is sufficiently bright (test of Step S26), i.e. above a lower image intensity threshold.

The exposure increments, may for example be constant fixed increments, or increments whose size varies. One example of a varying increment would be to follow a predefined progression such as increasing by a multiplying factor, e.g. 2 to double the exposure each time. Preferred values for the multiplying factor in these embodiments is between 1.5 and 2.5. Another example would be to vary the increment size interactively based on how far below the desired intensity level the last FISH probe image was determined to be, with the increments becoming smaller as the desired intensity level is approached, e.g. by using a multiplying factor that includes as a component the difference between the desired intensity and the current intensity.

With Step S29, the method concludes by saving a FISH probe image into a record. The preferable outcome is when the flow proceeds from Step S26 to Step S29, in which case the FISH probe image that is saved has an intensity value in the areas of interest that is of a desired value. The record preferably also saves the counterstain image. The FISH probe image that is saved may be the one most recently acquired, including the 'nul' dark image (in case of flow from Step S27 to Step S29) in the case the maximum exposure limit has been reached. Alternatively, the saved image might be an additional FISH probe image acquired as part of Step S29. Making an extra acquisition for the record may be to obtain an overall better quality image. For example, an exposure might be chosen that is deemed to be optimum based on a joint analysis of two or more of the FISH probe images acquired through different traversals of Step S24, e.g. an exposure value determined by interpolation of the image intensities of the most recent two or three iterations of the loop of Steps S24 to S28.

It is preferable if the processing of the images during the above acquisition process is done rapidly so that the method is acceptably quick, e.g. with the aid of a graphics processing unit (GPU). Moreover, the exposure increment ΔE is preferably set to a value that is small enough to provide fine adjustment, but not so small that the number of iterations makes the process as a whole unacceptably slow. In addition, it is noted that speed is the reason why the method starts with short exposures and increases them rather than the opposite, i.e. starting with long exposures and then shortening them.

The mask generated from the FISH probe image and the mask generated from the counterstain image may be generated using any combination of standard processing techniques, for example as described in the above-referenced text book chapters. The FISH probe images or the counterstain images may be color or grayscale. The images may be modified by applying a contrast enhancement filter. The mask generation may involve some segmentation to identify the areas of interest in the image. Segmentation may involve any or all of the following image processing techniques:
1. Variance based analysis to identify the seed areas
2. Adaptive thresholding
3. Morphological operations (as described above)
4. Contour identification
5. Contour merging based on proximity heuristic rules
6. Calculation of invariant image moments
7. Edge extraction (e.g. Sobel edge detection)
8. Curvature flow filtering
9. Superpixel clustering.

These image processing steps for generating the masks are described by way of example and should not be interpreted as being in any way limitative on the scope of the invention.

### COMPARATIVE EXAMPLES

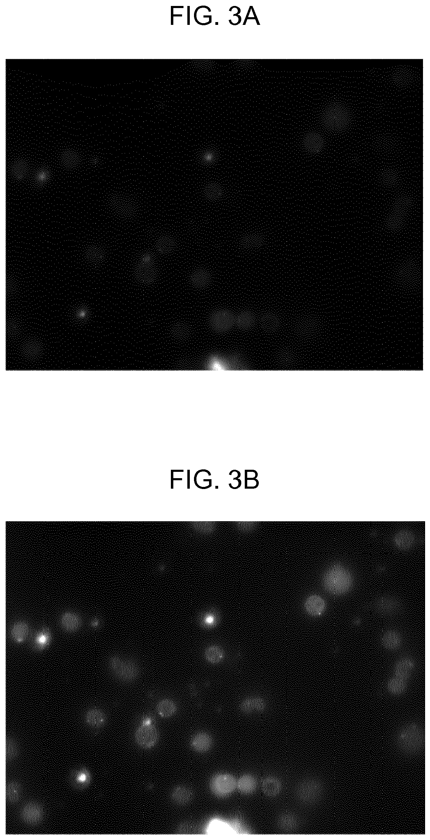

Figure 3A shows a FISH probe image taken without benefit of the invention. The bright fluorescent debris that is visible near the bottom edge of the image centrally from left to right has caused the auto-exposure method based on brightness across the whole image to underexpose the areas of interest, so they are too dark.

Figure 3B shows a FISH probe image of the same sample as Figure 3A taken with an optimized exposure according to an embodiment of the invention. The mask obtained from the FISH probe image has been used to exclude the brightly fluorescing debris from influencing the exposure, which is determined as described above. As a result, the areas of interest in the FISH probe image are appropriately exposed and the detail in the cell nuclei is visible.

### COMPUTING PLATFORMS AND NETWORK ENVIRONMENT

The proposed image processing may be carried out on a variety of computing architectures, in particular ones that are optimized for image processing, which may be based on central processing units (CPUs), GPUs, field-programmable gate arrays (FPGAs) and/or application-specific integrated circuits (ASICs). In some embodiments, the image processing software runs on Nvidia GPUs from Nvidia Corporation, Santa Clara, California, such as the Tesla K80 GPU. In other embodiments, the image processing software can run on generic CPUs. Faster processing may be obtainable by a purpose-designed processor for performing image processing calculations.

It will be understood that the computing power used for running the image processing software, whether it be based on CPUs, GPUs or some other processor type, may be hosted locally in a clinical network, e.g. the one described below, or remotely in a data center.

The proposed computer-automated method operates in the context of a laboratory information system (LIS) which in turn is typically part of a larger clinical network environment, such as a hospital information system (HIS) or picture archiving and communication system (PACS). In the LIS, the image data files will be retained in a database, typically a patient information database containing the electronic medical records of individual patients. The image data files will be taken from stained tissue samples mounted on slides, the slides bearing printed barcode labels by which the Image data files are tagged with suitable metadata, since the microscopes acquiring the Image data files are equipped with barcode readers. From a hardware perspective, the LIS will be a conventional computer network, such as a local area network (LAN) with wired and wireless connections as desired.

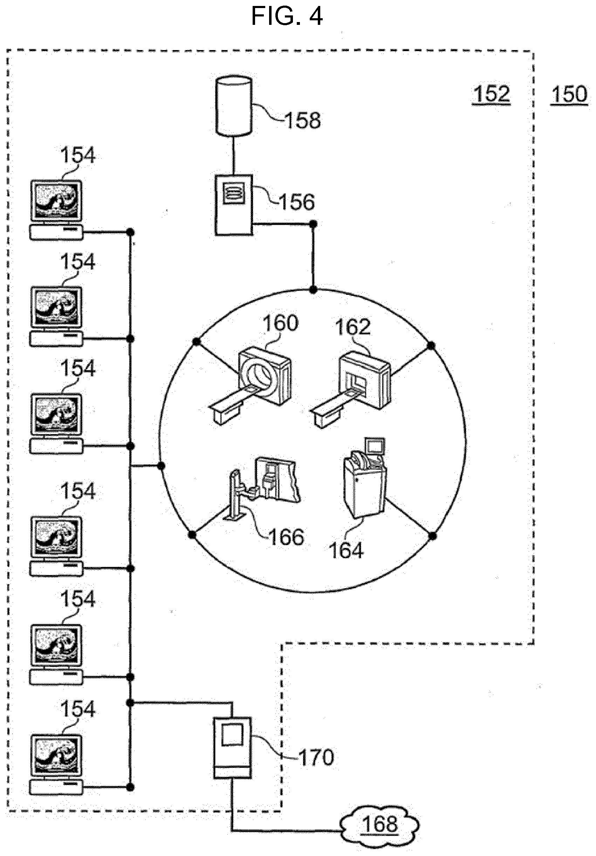

Figure 4 shows an example computer network which can be used in conjunction with embodiments of the invention. The network 150 comprises a LAN in a hospital 152. The hospital 152 is equipped with a number of workstations 154 which each have access, via the local area network, to a hospital computer server 156 having an associated storage device 158. A LIS, HIS or PACS archive is stored on the storage device 158 so that data in the archive can be accessed from any of the workstations 154. One or more of the workstations 154 has access to a graphics card and to software for computer-implementation of methods of generating images as described hereinbefore. The software may be stored locally at the or each workstation 154, or may be stored remotely and downloaded over the network 150 to a workstation 154 when needed. In other example, methods embodying the invention may be executed on the computer server with the workstations 154 operating as terminals. For example, the workstations may be configured to receive user input defining a desired FISH image data set and to display resulting images while image processing analysis is performed elsewhere in the system. Also, a number of FISH-image acquiring devices and other medical imaging devices 160, 162, 164, 166 are connected to the hospital computer server 156. Image data collected with the devices 160, 162, 164, 166 can be stored directly into the LIS, HIS or PACS archive on the storage device 156. Thus FISH images can be viewed and processed immediately after the corresponding FISH image data are recorded. The local area network is connected to the Internet 168 by a hospital Internet server 170, which allows remote access to the LIS, HIS or PACS archive. This is of use for remote accessing of the data and for transferring data between hospitals, for example, if a patient is moved, or to allow external research to be undertaken.

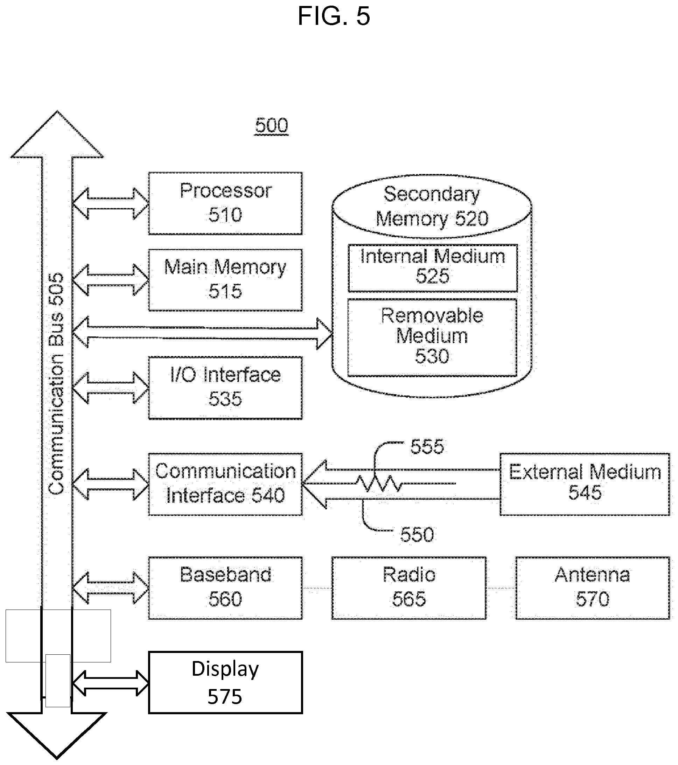

Figure 5 is a block diagram illustrating an example computing apparatus 500 that may be used in connection with various embodiments described herein. For example, computing apparatus 500 may be used as a computing node in the above-mentioned LIS or PACS system, for example a host computer from which processing of FISH images is carried out in conjunction with a suitable CPU or GPU.

Computing apparatus 500 can be a server or any conventional personal computer, or any other processor-enabled device that is capable of wired or wireless data communication. Other computing apparatus, systems and/or architectures may be also used, including devices that are not capable of wired or wireless data communication, as will be clear to those skilled in the art.

Computing apparatus 500 preferably includes one or more processors, such as processor 510. The processor 510 may be for example a CPU or GPU or arrays or combinations thereof such as CPU and GPU combinations. Additional processors may be provided, such as an auxiliary processor to manage input/output, an auxiliary processor to perform floating point mathematical operations such as a tensor processing unit (TPU), a special-purpose microprocessor having an architecture suitable for fast execution of signal processing algorithms (e.g., digital signal processor, image processor), a slave processor subordinate to the main processing system (e.g., back-end processor), an additional microprocessor or controller for dual or multiple processor systems, or a coprocessor. Such auxiliary processors may be discrete processors or may be integrated with the processor 510. Examples of CPUs which may be used with computing apparatus 500 are, the Pentium processor, Core i7 processor, and Xeon processor, all of which are available from Intel Corporation of Santa Clara, California. An example GPU which may be used with computing apparatus 500 is Tesla K80 GPU of Nvidia Corporation, Santa Clara, California.

Processor 510 is connected to a communication bus 505. Communication bus 505 may include a data channel for facilitating information transfer between storage and other peripheral components of computing apparatus 500. Communication bus 505 further may provide a set of signals used for communication with processor 510, including a data bus, address bus, and control bus (not shown). Communication bus 505 may comprise any standard or non-standard bus architecture such as, for example, bus architectures compliant with industry standard architecture (ISA), extended industry standard architecture (EISA), Micro Channel Architecture (MCA), peripheral component interconnect (PCI) local bus, or standards promulgated by the Institute of Electrical and Electronics Engineers (IEEE) including IEEE 488 general-purpose interface bus (GPIB), IEEE 696/S-100, and the like.

Computing apparatus 500 preferably includes a main memory 515 and may also include a secondary memory 520. Main memory 515 provides storage of instructions and data for programs executing on processor 510, such as one or more of the functions and/or modules discussed above. It should be understood that computer readable program instructions stored in the memory and executed by processor 510 may be assembler instructions, instruction-set-architecture (ISA) instructions, machine instructions, machine dependent instructions, microcode, firmware instructions, state-setting data, configuration data for integrated circuitry, or either source code or object code written in and/or compiled from any combination of one or more programming languages, including without limitation Smalltalk, C/C++, Java, JavaScript, Perl, Visual Basic, .NET, and the like. Main memory 515 is typically semiconductor-based memory such as dynamic random access memory (DRAM) and/or static random access memory (SRAM). Other semiconductor-based memory types include, for example, synchronous dynamic random access memory (SDRAM), Rambus dynamic random access memory (RDRAM), ferroelectric random access memory (FRAM), and the like, including read only memory (ROM).

The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Secondary memory 520 may optionally include an internal memory 525 and/or a removable medium 530. Removable medium 530 is read from and/or written to in any well-known manner. Removable storage medium 530 may be, for example, a magnetic tape drive, a compact disc (CD) drive, a digital versatile disc (DVD) drive, other optical drive, a flash memory drive, etc.

Removable storage medium 530 is a non-transitory computer-readable medium having stored thereon computer-executable code (i.e., software) and/or data. The computer software or data stored on removable storage medium 530 is read into computing apparatus 500 for execution by processor 510.

The secondary memory 520 may include other similar elements for allowing computer programs or other data or instructions to be loaded into computing apparatus 500. Such means may include, for example, an external storage medium 545 and a communication interface 540, which allows software and data to be transferred from external storage medium 545 to computing apparatus 500. Examples of external storage medium 545 may include an external hard disk drive, an external optical drive, an external magneto- optical drive, etc. Other examples of secondary memory 520 may include semiconductor- based memory such as programmable read-only memory (PROM), erasable programmable read-only memory (EPROM), electrically erasable read-only memory (EEPROM), or flash memory (block-oriented memory similar to EEPROM).

As mentioned above, computing apparatus 500 may include a communication interface 540. Communication interface 540 allows software and data to be transferred between computing apparatus 500 and external devices (e.g. printers), networks, or other information sourccs. For example, computer software or executable code may be transferred to computing apparatus 500 from a network server via communication interface 540. Examples of communication interface 540 include a built-in network adapter, network interface card (NIC), Personal Computer Memory Card International Association (PCMCIA) network card, card bus network adapter, wireless network adapter, Universal Serial Bus (USB) network adapter, modem, a network interface card (NIC), a wireless data card, a communications port, an infrared interface, an IEEE 1394 fire-wire, or any other device capable of interfacing system 550 with a network or another computing device. Communication interface 540 preferably implements industry-promulgated protocol standards, such as Ethernet IEEE 802 standards, Fiber Channel, digital subscriber line (DSL), asynchronous digital subscriber line (ADSL), frame relay, asynchronous transfer mode (ATM), integrated digital services network (ISDN), personal communications services (PCS), transmission control protocol/Internet protocol (TCP/IP), serial line Internet protocol/point to point protocol (SLIP/PPP), and so on, but may also implement customized or non-standard interface protocols as well.

Software and data transferred via communication interface 540 are generally in the form of electrical communication signals 555. These signals 555 may be provided to communication interface 540 via a communication channel 550. In an embodiment, communication channel 550 may be a wired or wireless network, or any variety of other communication links. Communication channel 550 carries signals 555 and can be implemented using a variety of wired or wireless communication means including wire or cable, fiber optics, conventional phone line, cellular phone link, wireless data communication link, radio frequency ("RF") link, or infrared link, just to name a few.

Computer-executable code (i.e., computer programs or software) is stored in main memory 515 and/or the secondary memory 520. Computer programs can also be received via communication interface 540 and stored in main memory 515 and/or secondary memory 520. Such computer programs, when executed, enable computing apparatus 500 to perform the various functions of the disclosed embodiments as described elsewhere herein.

In this document, the term "computer-readable medium" is used to refer to any non-transitory computer-readable storage media used to provide computer-executable code (e.g., software and computer programs) to computing apparatus 500. Examples of such media include main memory 515, secondary memory 520 (including internal memory 525, removable medium 530, and external storage medium 545), and any peripheral device communicatively coupled with communication interface 540 (including a network information server or other network device). These non-transitory computer-readable media are means for providing executable code, programming instructions, and software to computing apparatus 500. In an embodiment that is implemented using software, the software may be stored on a computer-readable medium and loaded into computing apparatus 500 by way of removable medium 530, I/O interface 535, or communication interface 540. In such an embodiment, the software is loaded into computing apparatus 500 in the form of electrical communication signals 555. The software, when executed by processor 510, preferably causes processor 510 to perform the features and functions described elsewhere herein.

I/O interface 535 provides an interface between one or more components of computing apparatus 500 and one or more input and/or output devices. Example input devices include, without limitation, keyboards, touch screens or other touch-sensitive devices, biometric sensing devices, computer mice, trackballs, pen-based pointing devices, and the like. Examples of output devices include, without limitation, cathode ray tubes (CRTs), plasma displays, light-emitting diode (LED) displays, liquid crystal displays (LCDs), printers, vacuum florescent displays (VFDs), surface-conduction electron-emitter displays (SEDs), field emission displays (FEDs), and the like.

Computing apparatus 500 also includes optional wireless communication components that facilitate wireless communication over a voice network and/or a data network. The wireless communication components comprise an antenna system 570, a radio system 565, and a baseband system 560. In computing apparatus 500, radio frequency (RF) signals are transmitted and received over the air by antenna system 570 under the management of radio system 565.

Antenna system 570 may comprise one or more antennae and one or more multiplexors (not shown) that perform a switching function to provide antenna system 570 with transmit and receive signal paths. In the receive path, received RF signals can be coupled from a multiplexor to a low noise amplifier (not shown) that amplifies the received RF signal and sends the amplified signal to radio system 565.

Radio system 565 may comprise one or more radios that are configured to communicate over various frequencies. In an embodiment, radio system 565 may combine a demodulator (not shown) and modulator (not shown) in one integrated circuit (IC). The demodulator and modulator can also be separate components. In the incoming path, the demodulator strips away the RF carrier signal leaving a baseband receive audio signal, which is sent from radio system 565 to baseband system 560.

If the received signal contains audio information, then baseband system 560 decodes the signal and converts it to an analog signal. Then the signal is amplified and sent to a speaker. Baseband system 560 also receives analog audio signals from a microphone. These analog audio signals are converted to digital signals and encoded by baseband system 560. Baseband system 560 also codes the digital signals for transmission and generates a baseband transmit audio signal that is routed to the modulator portion of radio system 565. The modulator mixes the baseband transmit audio signal with an RF carrier signal generating an RF transmit signal that is routed to antenna system 570 and may pass through a power amplifier (not shown). The power amplifier amplifies the RF transmit signal and routes it to antenna system 570 where the signal is switched to the antenna port for transmission.

Baseband system 560 is also communicatively coupled with processor 510, which may be a CPU. Processor 510 has access to data storage areas 515 and 520. Processor 510 is preferably configured to execute instructions (i.e., computer programs or software) that can be stored in main memory 515 or secondary memory 520. Computer programs can also be received from baseband processor 560 and stored in main memory 510 or in secondary memory 520, or executed upon receipt. Such computer programs, when executed, enable computing apparatus 500 to perform the various functions of the disclosed embodiments. For example, data storage areas 515 or 520 may include various software modules.

The computing apparatus further comprises a display 575 directly attached to the communication bus 505 which may be provided instead of or addition to any display connected to the I/O interface 535 referred to above.

Various embodiments may also be implemented primarily in hardware using, for example, components such as application specific integrated circuits (ASICs), programmable logic arrays (PLA), or field programmable gate arrays (FPGAs). Implementation of a hardware state machine capable of performing the functions described herein will also be apparent to those skilled in the relevant art. Various embodiments may also be implemented using a combination of both hardware and software.

Furthermore, those of skill in the art will appreciate that the various illustrative logical blocks, modules, circuits, and method steps described in connection with the above described figures and the embodiments disclosed herein can often be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled persons can implement the described functionality in varying ways for each particular application, but such implementation decisions should not be interpreted as causing a departure from the scope of the invention. In addition, the grouping of functions within a module, block, circuit, or step is for ease of description. Specific functions or steps can be moved from one module, block, or circuit to another without departing from the invention.

Moreover, the various illustrative logical blocks, modules, functions, and methods described in connection with the embodiments disclosed herein can be implemented or performed with a general purpose processor, a digital signal processor (DSP), an ASIC, FPGA, or other programmable logic device, discrete gate or transistor logic, discrete hardware components, or any combination thereof designed to perform the functions described herein. A general-purpose processor can be a microprocessor, but in the alternative, the processor can be any processor, controller, microcontroller, or state machine. A processor can also be implemented as a combination of computing devices, for example, a combination of a DSP and a microprocessor, a plurality of microprocessors, one or more microprocessors in conjunction with a DSP core, or any other such configuration.

Additionally, the steps of a method or algorithm described in connection with the embodiments disclosed herein can be embodied directly in hardware, in a software module executed by a processor, or in a combination of the two. A software module can reside in RAM memory, flash memory, ROM memory, EPROM memory, EEPROM memory, registers, hard disk, a removable disk, a CD-ROM, or any other form of storage medium including a network storage medium. An exemplary storage medium can be coupled to the processor such that the processor can read information from, and write information to, the storage medium. In the alternative, the storage medium can be integral to the processor. The processor and the storage medium can also reside in an ASIC.

A computer readable storage medium, as referred to herein, is not to be construed as being transitory signals *per se,* such as radio waves or other freely propagating electromagnetic waves, electromagnetic waves propagating through a waveguide or other transmission media (e.g., light pulses passing through a fiber-optic cable), or electrical signals transmitted through a wire.

Any of the software components described herein may take a variety of forms. For example, a component may be a stand-alone software package, or it may be a software package incorporated as a "tool" in a larger software product. It may be downloadable from a network, for example, a website, as a stand-alone product or as an add-in package for installation in an existing software application. It may also be available as a client- server software application, as a web-enabled software application, and/or as a mobile application.

Embodiments of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

The computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The illustrated flowcharts and block diagrams illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

It will be clear to one skilled in the art that many improvements and modifications can be made to the foregoing exemplary embodiment without departing from the scope of the present invention as defined in the appended claims.

## Claims

1. A method of controlling an image acquisition apparatus (5) comprising a fluorescence microscope arrangement to acquire a fluorescence *in situ* hybridization, FISH, probe image of a sample labeled with a FISH probe, the method comprising:
setting an exposure for acquiring a FISH probe image to an initial value, wherein the initial exposure value is set so as to acquire an underexposed FISH probe image (S23);
acquiring a FISH probe image with the set value of the exposure (S24);
image processing the FISH probe image by applying at least one morphological operator to identify areas of interest around locations of elevated image intensity, and then generating a FISH probe image mask for filtering out all but the areas of interest (S25);
applying the mask to the FISH probe image to determine image intensity in the areas of interest (S25);
determining if the image intensity has a desired intensity value which indicates correct exposure of the FISH probe image (S26), and if 'yes'
then generating and saving a FISH record including a correctly exposed FISH probe image (S29), and if 'no'
then adjusting the exposure value (S28), wherein adjusting the exposure value consists of increasing its value, and iterating the method with the adjusted value of the exposure so that the exposure value is successively increased from the initial value as the method iterates.

2. The method of claim 1, wherein the successive increasing of the exposure is terminated once a maximum exposure value is reached without obtaining a FISH probe image with an image intensity of the desired intensity value, in which case the FISH record is generated and saved with one of the acquired FISH probe images and metadata indicating that the FISH probe image has insufficient FISH signal (S27).

3. The method of claim 1, wherein determining the adjusted value of the exposure comprises multiplying a first factor based on the ratio between the exposure and the image intensity of the FISH probe image with a second factor based on the desired image intensity.

4. The method of claim 1, wherein the method is iterated at least once to acquire a plurality of FISH probe images, and wherein determining the adjusted value of the exposure comprises multiplying a first factor based on a sum of the ratios of the exposures and the image intensities of each of the plurality of FISH probe images obtained with a second factor based on the desired image intensity.

5. The method of claim 1, wherein the method is iterated at least once to acquire a plurality of FISH probe images, and wherein determining the adjusted value of the exposure comprises multiplying a first factor based on the ratio between the exposure and the image intensity of one of the subsequent FISH probe images with a second factor based on the desired image intensity.

6. The method of claim 1, wherein the sample is additionally labeled with a counterstain, the method further comprising:
acquiring a counterstain image of the sample (S21);
image processing the counterstain image to generate a counterstain image mask that filters out all but areas of or around the counterstained areas (S22); and
confining the image processing of the FISH probe image to the areas not filtered out by the counterstain image mask (S25).

7. The method of claim 6, wherein the FISH record further includes the counterstain image.

8. The method of claim 6 or 7, wherein the counterstain is configured to stain cell nuclei.

9. A computer program product for controlling an image acquisition apparatus to acquire a fluorescence *in situ* hybridization, FISH, probe image of a sample labeled with a FISH probe, the computer program product bearing machine readable instructions for performing the method of any one of claims 1 to 8.

10. An image acquisition apparatus (5) for acquiring a fluorescence *in situ* hybridization, FISH, probe image of a sample labeled with a FISH probe, the apparatus comprising:
a fluorescence microscope arrangement operable to acquire FISH probe images of a sample; and
a control computer (35) configured to determine appropriate exposures for acquiring FISH probe images with the fluorescence microscope arrangement based on a method of:
setting an exposure for acquiring a FISH probe image to an initial value, wherein the initial exposure value is set so as to acquire an underexposed FISH probe image (S23);
acquiring a FISH probe image with the set value of the exposure (S24);
image processing the FISH probe image by applying at least one morphological operator to identify areas of interest around locations of elevated image intensity, and then generating a FISH probe image mask for filtering out all but the areas of interest (S25);
applying the mask to the FISH probe image to determine image intensity in the areas of interest (S25);
determining if the image intensity has a desired intensity value which indicates correct exposure of the FISH probe image (S26), and if 'yes'
then generating and saving a FISH record including a correctly exposed FISH probe image (S29), and if 'no'
then adjusting the exposure value (S28), wherein adjusting the exposure value consists of increasing its value, and iterating the method with the adjusted value of the exposure so that the exposure value is successively increased from the initial value as the method iterates.

11. The apparatus of claim 10, wherein for samples additionally labeled with a counterstain the control computer is further configured to determine the appropriate exposures by additionally:
acquiring a counterstain image of the sample (S21);
image processing the counterstain image to generate a counterstain image mask that filters out all but areas of or around the counterstained areas (S22); and
confining the image processing of the FISH probe image to the areas not filtered out by the counterstain image mask (S25).

12. The apparatus of claim 10 or 11, further comprising:
a display (575); and
a display output operable to access the FISH record and transmit the FISH probe image to the display such that the FISH probe image is displayed.

13. A network (15) comprising:
a computer (154);
a data repository (156, 158) configured to store FISH records, the FISH records including FISH probe images;
network connections enabling transfer of the FISH records or parts thereof between the computer and the data repository; and
an image acquisition apparatus (5; 164) according to any of claims 10 to 12 operable to generate FISH records and save them to the data repository.

## Patentansprüche

1. Verfahren zum Steuern einer Bilderfassungsvorrichtung (5), die eine Fluoreszenzmikroskopanordnung zur Erfassung eines Fluoreszenz-*in-situ*-Hybridisierungs-(FISH)-Sondenbildes einer mit einer FISH-Sonde markierten Probe umfasst, wobei das Verfahren Folgendes beinhaltet:
Einstellen einer Belichtung zum Erfassen eines FISH-Sondenbildes auf einen Anfangswert, wobei der Belichtungsanfangswert so eingestellt wird, dass ein unterbelichtetes FISH-Sondenbild erfasst wird (S23);
Erfassen eines FISH-Sondenbildes mit dem eingestellten Belichtungswert (S24);
Unterziehen des FISH-Sondenbildes einer Bildverarbeitung durch Anwenden wenigstens eines morphologischen Operators, um Bereiche von Interesse um Orte mit erhöhter Bildintensität zu identifizieren, und dann Erzeugen einer FISH-Sondenbildmaske zum Herausfiltern aller Bereiche außer den Bereichen von Interesse (S25);
Anwenden der Maske auf das FISH-Sondenbild, um die Bildintensität in den Bereichen von Interesse zu bestimmen (S25);
Feststellen, ob die Bildintensität einen gewünschten Intensitätswert hat, der die korrekte Belichtung des FISH-Sondenbildes anzeigt (S26), und wenn 'ja'
Erzeugen und Speichern eines FISH-Datensatzes, der ein korrekt belichtetes FISH-Sondenbild beinhaltet (S29), und wenn 'nein',
Einstellen des Belichtungswertes (S28), wobei das Einstellen des Belichtungswertes darin besteht, den Wert zu erhöhen, und Wiederholen des Verfahrens mit dem eingestellten Belichtungswert, so dass der Belichtungswert sukzessiv ab dem Anfangswert erhöht wird, während das Verfahren wiederholt wird.

2. Verfahren nach Anspruch 1, wobei das sukzessive Erhöhen der Belichtung beendet wird, sobald ein maximaler Belichtungswert erreicht ist, ohne dass ein FISH-Sondenbild mit einer Bildintensität des gewünschten Intensitätswertes erhalten wird, wobei in diesem Fall der FISH-Datensatz erzeugt und mit einem der erfassten FISH-Sondenbilder und Metadaten gespeichert wird, so dass angezeigt wird, dass das FISH-Sondenbild ein unzureichendes FISH-Signal aufweist (S27).

3. Verfahren nach Anspruch 1, wobei das Bestimmen des eingestellten Belichtungswertes das Multiplizieren eines ersten Faktors auf der Basis des Verhältnisses zwischen der Belichtung und der Bildintensität des FISH-Sondenbildes mit einem zweiten Faktor auf der Basis der gewünschten Bildintensität beinhaltet.

4. Verfahren nach Anspruch 1, wobei das Verfahren wenigstens einmal wiederholt wird, um eine Mehrzahl von FISH-Sondenbildern zu erfassen, und wobei das Bestimmen des eingestellten Belichtungswertes das Multiplizieren eines ersten Faktors auf der Basis einer Summe der Verhältnisse der Belichtungen und der Bildintensitäten von jedem der Mehrzahl von erhaltenen FISH-Sondenbildern mit einem zweiten Faktor auf der Basis der gewünschten Bildintensität beinhaltet.

5. Verfahren nach Anspruch 1, wobei das Verfahren wenigstens einmal wiederholt wird, um eine Mehrzahl von FISH-Sondenbildern zu erfassen, und wobei das Bestimmen des eingestellten Belichtungswertes durch das Multiplizieren eines ersten Faktors auf der Basis des Verhältnisses zwischen Belichtung und Bildintensität von einem der nachfolgenden FISH-Sondenbilder mit einem zweiten Faktor auf der Basis der gewünschten Bildintensität beinhaltet.

6. Verfahren nach Anspruch 1, wobei die Probe zusätzlich mit einer Gegenfärbung markiert wird, wobei das Verfahren ferner Folgendes beinhaltet:
Erfassen eines Gegenfärbungsbildes der Probe (S21);
Unterziehen des Gegenfärbungsbildes einer Bildverarbeitung, um eine Gegenfärbungsbildmaske zu erzeugen, die alle außer den gegengefärbten Bereichen oder den darum herum liegenden Bereichen herausfiltert (S22); und
Beschränken der Bildverarbeitung des FISH-Sondenbildes auf die Bereiche, die von der Gegenfärbungsbildmaske nicht herausgefiltert wurden (S25).

7. Verfahren nach Anspruch 6, wobei der FISH-Datensatz ferner das Gegenfärbungsbild beinhaltet.

8. Verfahren nach Anspruch 6 oder 7, wobei die Gegenfärbung so konfiguriert ist, dass sie Zellkerne färbt.

9. Computerprogrammprodukt zur Steuerung einer Bilderfassungsvorrichtung zur Erfassung eines Fluoreszenz-*in-situ*-Hybridisierungs-(FISH)-Sondenbildes einer mit einer FISH-Sonde markierten Probe, wobei das Computerprogrammprodukt maschinenlesbare Anweisungen zur Durchführung des Verfahrens nach einem der Ansprüche 1 bis 8 enthält.

10. Bilderfassungsvorrichtung (5) zum Erfassen eines Fluoreszenz-*in-situ-*Hybridisierungs-(FISH)-Sondenbildes einer mit einer FISH-Sonde markierten Probe, wobei die Vorrichtung Folgendes umfasst:
eine Fluoreszenzmikroskopanordnung mit der Aufgabe, FISH-Sondenbildern einer Probe zu erfassen; und
einen Steuercomputer (35), der so konfiguriert ist, dass er geeignete Belichtungen zur Erfassung von FISH-Sondenbildern mit der Fluoreszenzmikroskopanordnung bestimmt, basierend auf einem Verfahren, das Folgendes beinhaltet:
Einstellen einer Belichtung zum Erfassen eines FISH-Sondenbildes auf einen Anfangswert, wobei der Belichtungsanfangswert so eingestellt wird, dass ein unterbelichtetes FISH-Sondenbild erfasst wird (S23);
Erfassen eines FISH-Sondenbildes mit dem eingestellten Belichtungswert (S24);
Unterziehen des FISH-Sondenbildes einer Bildverarbeitung durch Anwenden wenigstens eines morphologischen Operators, um Bereiche von Interesse um Orte mit erhöhter Bildintensität zu identifizieren, und dann Erzeugen einer FISH-Sondenbildmaske zum Herausfiltern aller Bereiche außer den Bereichen von Interesse (S25);
Anwenden der Maske auf das FISH-Sondenbild, um die Bildintensität in den Bereichen von Interesse zu bestimmen (S25);
Feststellen, ob die Bildintensität einen gewünschten Intensitätswert hat, der die korrekte Belichtung des FISH-Sondenbildes anzeigt (S26), und wenn 'ja'
Erzeugen und Speichern eines FISH-Datensatzes, der ein korrekt belichtetes FISH-Sondenbild beinhaltet (S29), und wenn 'nein',
Einstellen des Belichtungswertes (S28), wobei das Einstellen des Belichtungswertes darin besteht, den Wert zu erhöhen, und Wiederholen des Verfahrens mit dem eingestellten Belichtungswert, so dass der Belichtungswert sukzessiv ab dem Anfangswert erhöht wird, während das Verfahren wiederholt wird.

11. Vorrichtung nach Anspruch 10, wobei für Proben, die zusätzlich mit einer Gegenfärbung markiert wurden, der Steuercomputer ferner so konfiguriert ist, dass er die geeigneten Belichtungen bestimmt, durch zusätzlich:
Erfassen eines Gegenfärbungsbildes der Probe (S21);
Unterziehen des Gegenfärbungsbildes einer Bildverarbeitung, um eine Gegenfärbungsbildmaske zu erzeugen, die alle außer den gegengefärbten Bereichen oder den darum herum liegenden Bereiche herausfiltert (S22); und
Beschränken der Bildverarbeitung des FISH-Sondenbildes auf die Bereiche, die von der Gegenfärbungsbildmaske nicht herausgefiltert wurden (S25).

12. Vorrichtung nach Anspruch 10 oder 11, die ferner Folgendes umfasst:
ein Display (575); und
einen Displayausgang mit der Aufgabe, auf den FISH-Datensatz zuzugreifen und das FISH-Sondenbild zum Display zu übertragen, so dass das FISH-Sondenbild angezeigt wird.

13. Netzwerk (15), das Folgendes umfasst:
einen Computer (154);
einen Datenspeicher (156, 158), der so konfiguriert ist, dass er FISH-Datensätze speichert, wobei die FISH-Datensätze FISH-Sondenbilder enthalten;
Netzwerkverbindungen, die die Übertragung der FISH-Datensätze oder Teile davon zwischen dem Computer und dem Datenspeicher ermöglichen; und
eine Bilderfassungsvorrichtung (5; 164) nach einem der Ansprüche 10 bis 12 mit der Aufgabe, FISH-Datensätze zu erzeugen und sie im Datenspeicher zu speichern.

## Revendications

1. Procédé de commande d'un appareil d'acquisition d'images (5) comprenant un agencement de microscope à fluorescence pour acquérir une image sonde d'hybridation *in situ* en fluorescence, FISH, d'un échantillon étiqueté avec une sonde FISH, le procédé comprenant :
le réglage à une valeur initiale d'une exposition pour acquérir une image sonde FISH, la valeur d'exposition initiale étant réglée de manière à acquérir une image sonde FISH sous-exposée (S23) ;
l'acquisition d'une image sonde FISH avec la valeur d'exposition réglée (S24) ;
le traitement d'image de l'image sonde FISH en appliquant au moins un opérateur morphologique pour identifier des zones d'intérêt autour d'endroits d'une intensité d'image élevée, puis en générant un masque d'image sonde FISH pour ne conserver par filtrage que les zones d'intérêt (S25) ;
l'application du masque à l'image sonde FISH pour déterminer une intensité d'image dans les zones d'intérêt (S25) ;
la détermination que l'intensité d'image possède ou non une valeur d'intensité souhaitée qui indique une exposition correcte de l'image sonde FISH (S26), et dans l'affirmative
la génération et la sauvegarde d'un enregistrement FISH comportant une image sonde FISH correctement exposée (S29), et dans la négative
l'ajustement de la valeur d'exposition (S28), l'ajustement de la valeur d'exposition consistant à augmenter sa valeur, et la répétition du procédé avec la valeur ajustée de l'exposition de telle sorte que la valeur d'exposition soit augmentée successivement à partir de la valeur initiale au fur et à mesure des répétitions du procédé.

2. Procédé selon la revendication 1, dans lequel l'augmentation successive de l'exposition est arrêtée une fois qu'une valeur d'exposition maximale est atteinte sans obtenir une image sonde FISH d'une intensité d'image de la valeur d'intensité souhaitée, auquel cas l'enregistrement FISH est généré et sauvegardé avec une des images sondes FISH acquises et des métadonnées indiquant que l'image sonde FISH présente un signal FISH insuffisant (S27).

3. Procédé selon la revendication 1, dans lequel la détermination de la valeur ajustée de l'exposition comprend la multiplication d'un premier facteur basé sur le rapport entre l'exposition et l'intensité d'image de l'image sonde FISH avec un second facteur basé sur l'intensité d'image souhaitée.

4. Procédé selon la revendication 1, le procédé étant répété au moins une fois pour acquérir une pluralité d'images sondes FISH, et dans lequel la détermination de la valeur ajustée de l'exposition comprend la multiplication d'un premier facteur basé sur une somme des rapports des expositions et des intensités d'images de chacune de la pluralité d'images sondes FISH obtenues avec un second facteur basé sur l'intensité d'image souhaitée.

5. Procédé selon la revendication 1, le procédé étant répété au moins une fois pour acquérir une pluralité d'images sondes FISH, et dans lequel la détermination de la valeur ajustée de l'exposition par multiplication d'un premier facteur basé sur le rapport entre l'exposition et l'intensité d'image d'une des images sondes FISH suivantes obtenues avec un second facteur basé sur l'intensité d'image souhaitée.

6. Procédé selon la revendication 1, dans lequel l'échantillon est de plus étiqueté avec un contre-colorant, le procédé comprenant en outre :
l'acquisition d'une image à contre-colorant de l'échantillon (S21) ;
le traitement d'image de l'image à contre-colorant pour générer un masque d'image à contre-colorant qui ne conserve par filtrage que les zones à contre-colorant ou des zones autour de celles-ci (S22) ; et
le confinement du traitement d'image de l'image sonde FISH aux zones conservées par filtrage par le masque d'image à contre-colorant (S25).

7. Procédé selon la revendication 6, dans lequel l'enregistrement FISH comporte en outre l'image à contre-colorant.

8. Procédé selon la revendication 6 ou 7, dans lequel le contre-colorant est configuré pour colorer des noyaux de cellules.

9. Produit-programme informatique pour commander un appareil d'acquisition d'images afin d'acquérir une image sonde d'hybridation *in situ* en fluorescence, FISH, d'un échantillon étiqueté avec une sonde FISH, le produit-programme informatique comportant des instructions lisibles par machine pour réaliser le procédé selon l'une quelconque des revendications 1 à 8.

10. Appareil d'acquisition d'images (5) pour acquérir une image sonde d'hybridation *in situ* en fluorescence, FISH, d'un échantillon étiqueté avec une sonde FISH, l'appareil comprenant :
un agencement de microscope à fluorescence exploitable pour acquérir des images sondes FISH d'un échantillon ; et
un ordinateur de commande (35) configuré pour déterminer des expositions appropriées afin d'acquérir des images sondes FISH avec l'agencement de microscope à fluorescence selon un procédé de :
réglage à une valeur initiale d'une exposition pour acquérir une image sonde FISH, la valeur d'exposition initiale étant réglée de manière à acquérir une image sonde FISH sous-exposée (S23) ;
acquisition d'une image sonde FISH avec la valeur d'exposition réglée (S24) ;
traitement d'image de l'image sonde FISH en appliquant au moins un opérateur morphologique pour identifier des zones d'intérêt autour d'endroits d'une intensité d'image élevée, puis en générant un masque d'image sonde FISH pour ne conserver par filtrage que les zones d'intérêt (S25) ;
application du masque à l'image sonde FISH pour déterminer une intensité d'image dans les zones d'intérêt (S25) ;
détermination que l'intensité d'image possède ou non une valeur d'intensité souhaitée qui indique une exposition correcte de l'image sonde FISH (S26), et dans l'affirmative
génération et sauvegarde d'un enregistrement FISH comportant une image sonde FISH correctement exposée (S29), et dans la négative
ajustement de la valeur d'exposition (S28), l'ajustement de la valeur d'exposition consistant à augmenter sa valeur, et répétition du procédé avec la valeur ajustée de l'exposition de telle sorte que la valeur d'exposition soit augmentée successivement à partir de la valeur initiale au fur et à mesure des répétitions du procédé.

11. Appareil selon la revendication 10, dans lequel pour des échantillons étiquetés de plus avec un contre-colorant l'ordinateur de commande est configuré en outre pour déterminer les expositions appropriées en de plus :
acquérant une image à contre-colorant de l'échantillon (S21) ;
réalisant un traitement d'image de l'image à contre-colorant pour générer un masque d'image à contre-colorant qui ne conserve par filtrage que les zones à contre-colorant ou des zones autour de celles-ci (S22) ; et
confinant le traitement d'image de l'image sonde FISH aux zones conservées par filtrage par le masque d'image à contre-colorant (S25).

12. Appareil selon la revendication 10 ou 11, comprenant en outre :
un afficheur (575) ; et
une sortie d'afficheur exploitable pour accéder à l'enregistrement FISH et transmettre l'image sonde FISH à l'afficheur de manière à afficher l'image sonde FISH.

13. Réseau (15) comprenant :
un ordinateur (154) ;
un référentiel de données (156, 158) configuré pour stocker des enregistrements FISH, les enregistrements FISH comportant des images sondes FISH ;
des connexions de réseau permettant le transfert des enregistrements FISH ou de parties de ceux-ci entre l'ordinateur et le référentiel de données ; et
un appareil d'acquisition d'images (5 ; 164) selon l'une quelconque des revendications 10 à 12 exploitable pour générer des enregistrements FISH et les sauvegarder dans le référentiel de données.
